# EUROPEAN PATENT APPLICATION

(11) **EP 4 667 450 A1**
(43) Date of publication of application: **24.12.2025**
(21) Application number: 24183290.6
(22) Date of filing: 20.06.2024
(51) Int. Cl.: C07C 29/76, C07C 29/80, C07C 31/20

(54) **A METHOD OF EXTRACTING ETHYLENE GLYCOL FROM A LIQUID SOLUTION**

(71) Applicant: DePoly SA, 1950 Sion (CH)
(72) Inventor: Hlaiem, Mohamed, 3008 Bern (CH)
(74) Representative: Prins Intellectual Property AG

(57) **Abstract**

A method of extracting ethylene glycol from an initial liquid solution comprising water, ethanol, ethylene glycol (EG), and at least one salt is described, the method comprising the steps of: a. removing the at least one salt from the initial liquid solution using an ion exchange resin obtaining a first solution; b. performing a first distillation step in a first distillation column with the first solution at a temperature between 100-180 °C extracting water and ethanol on top of the first distillation column and obtaining a second solution containing ethylene glycol and the rest of the first solution on the bottom of the first distillation column; and c. performing a second distillation step with the second solution containing ethylene glycol at a temperature between 200-290 °C obtaining purified ethylene glycol on the top of the second distillation column and the rest of the second solution on the bottom of the second distillation column.

## Description

### Technical Field

The invention relates to a method of extracting ethylene glycol from a liquid solution comprising water, ethanol, ethylene glycol and at least one salt.

### Technical Background

The recycling of polymer and plastic materials is a challenge and has become increasingly important to manage plastic waste and minimize its impact on humans and the environment. In addition, it is desired to separate the polymers into its monomers and to reuse the monomers. WO2020173961A1 provides a method of alkaline hydrolysis of one or more plastic polymers into terephthalic acid (TPA) and/or ethylene glycol (EG) and/or other monomers that form the one or more plastic polymers, the method comprising: a) contacting the one or more plastic polymers with a metal oxide in a solution in the presence of a base to provide a reaction mixture; b) stirring the reaction mixture during appropriate time under UV light; c) recovering terephthalic acid, ethylene glycol and/or the other monomers from the reaction mixture.

The depolymerization reaction of WO2020173961A1 leads to two phases, a liquid and a solid phase. The liquid phase comprises a mixture of water, ethanol, ethylene glycol, and other trace elements and compounds, such as dyes and salt. This liquid mixture may need to be partially separated by removal of one component, such as ethylene glycol, or it may be fully separated before further use of the components of the mixture. For the extraction and purification of ethylene glycol form the mixture, the presence of salt, such as sodium hydroxide, is a major problem when it comes to removing water from that solution. Current methods use distillation for extracting ethylene glycol from a liquid solution. By evaporating water, the salt such as sodium hydroxide precipitates and forms solids. These solids could damage the distillation column by blockage, resulting in many problems such as handling of the heavy part of the products, heat exchange efficiency, etc. The challenge becomes more difficult when the ethylene glycol amount is very low, making it hard to extract from the solids, when they form in the distillation step, because the ethylene glycol molecules go inside the particles of the solid salt.

Another problem is the presence of dyes in the liquid solution when extracting ethylene glycol. In the distillation column, the presence of dyes could form layers around the column, especially while working on high temperature, reducing the efficiency of the distillation.

Current methods of separating, extracting and purifying ethylene glycol from a mixture of water, ethanol, ethylene glycol are energy intensive, especially when the mixture contains high concentration of dissolved salt, and therefore lead to relatively high cost.

Accordingly, there is a need for an improved, simplified and cost-effective method for extracting and purifying ethylene glycol from a liquid solution comprising a mixture of water, ethanol, ethylene glycol, at least one salt and further components, in order to achieve high quantity and high quality of ethylene glycol such as the virgin ethylene glycol, which can be reused. The ethylene glycol can be reused for many applications such as, for example, PET polymerisation as a basic monomer or as an additive as an antifreeze agent.

### Summary of the Invention

It is an objective of the invention to provide an improved, simplified and cost-effective method for extracting and purifying ethylene glycol from a liquid solution comprising at least one salt and to achieve high quantity and high quality of ethylene glycol, which can be used as the virgin ethylene glycol for many polymerisation processes or for example as an additive such as an antifreeze agent.

At least one of the objectives of the present invention is achieved by the method of extracting ethylene glycol (EG) according to claim 1.

The method of extracting ethylene glycol from an initial liquid solution comprising water, ethanol, ethylene glycol (EG), and at least one salt, comprises the steps of:
a. removing the at least one salt from the initial liquid solution using an ion exchange resin obtaining a first solution;
b. performing a first distillation step in a first distillation column with the first solution at a temperature between 100-180 °C, extracting water and ethanol on top of the first distillation column and obtaining a second solution containing ethylene glycol and the rest of the first solution on the bottom of the first distillation column; and
c. performing a second distillation step with the second solution containing ethylene glycol at a temperature between 200-290 °C obtaining purified ethylene glycol on the top of the second distillation column and the rest of the second solution on the bottom of the second distillation column.

When recycling polymers, it is desired to separate and extract the respective monomers and purify them for reuse. The polymers to be recycled and depolymerized usually come from a waste source of plastic material. A depolymerization process is described in PCT Application Publication No. WO2020173961A1 which leads to a liquid phase comprising a mixture of water, ethanol, ethylene glycol, and other contaminants, trace elements and compounds, such as dyes and salt. The inventive method of the present invention may be used to extract and separate ethylene glycol from such a liquid phase comprising water-ethanol-ethylene glycol as described in WO2020173964A1 but may also be used to extract ethylene glycol from water-ethanol-ethylene glycol mixtures from other sources.

The initial liquid solution of the present invention comprises water, ethanol, ethylene glycol, at least one salt and usually at least one contaminant. Preferably, the initial liquid solution originates from a waste source such as the liquid solution coming from a PET depolymerization process, as for example as described in WO2020173964A1.

The first important step of the present inventive method is the removal of the salt from the initial liquid solution before going through distillation. The at least one salt present in the initial liquid solution is a water-soluble sodium salt, potassium salt, calcium salt or magnesium salt. Preferably, the at least one salt is sodium hydroxide (NaOH). The amount of salt in the initial liquid solution coming e.g. from a depolymerization process is usually quite high. The presence of the salt would damage the distillation column in a distillation process while evaporating water and ethanol in a first step. In addition, the ethylene glycol amount is very low, and the ethylene glycol molecules would go inside the particles of the solid salt, when they form in the first distillation step, making it hard to extract the ethylene glycol from the solids. Thus, it is important to remove the salt before starting with the first distillation step.

According to the method of the present invention, in a first step (a) the initial liquid solution is pretreated with an ion exchange resin to remove the salt, preferably the sodium ion, from the initial liquid solution. The ion exchange resin is added to the initial liquid solution and reacts with the salt ion of the initial liquid solution. Ion exchange resins are known to be used to remove dissolved salts, targeting either the cation or the anion forming the salt, when the concentration of the salt is very low, for example for water purification. It was not expected that ion exchange resins, as shown in the present invention, can efficiently be used to remove salt in high concentrations before distillation. Especially, it was not expected that, according to the present invention, the ion exchange resin is able to remove the sodium ion, as the small sodium cation is known to be challenging to remove at high concentration. Industrially, reverse osmosis is used to remove the sodium ion like in the case of water desalination, which is an expensive process.

The removal of the salt results in either a neutralized or acidic first solution, depending on the salt present in the initial liquid solution. The first solution obtained after the removal of the salt contains water, ethanol, ethylene glycol and the rest of the first solution, usually contaminants. If the salt present in the initial liquid solution is for example sodium hydroxide, calcium hydroxide or potassium hydroxide, the removal of the salt results in neutralization of the initial liquid solution from an initial pH of 14 to a pH of 6 to 7, obtaining a first solution, also called neutralized solution, which contains water, ethanol, ethylene glycol and usually contaminants.

Preferably, the ion exchange resin is a strong cationic resin, preferably sulfonic acidic resin. The strong cationic resin reacts with the salt ion and produces water.

After the removal of the salt in the initial liquid solution, a first distillation step (b) is performed in a first distillation column with the first solution at a temperature between 100-180°C, preferably between at 110-120°C, wherein water and ethanol are evaporated and a second solution containing ethylene glycol and the rest of the first solution, such as for example contaminants, is obtained on the bottom of the first distillation column. The rest of the first solution after the first distillation step (b) contains anything that is left of the first solution after the evaporation of water and ethanol and next to ethylene glycol. Thus, the rest of the first solution usually contains contaminants, and the second solution contains ethylene glycol and the rest of the first solution, usually ethylene glycol and contaminants.

Then, the second solution is injected into a second distillation column and a second distillation step (c) is performed with the second solution containing ethylene glycol and the rest of the first solution at a temperature between 200-290°C, preferably at 210-290°C, obtaining purified ethylene glycol on the top of the second distillation column and the rest of the second solution on the bottom of the second distillation column.

The second distillation column provides on top the evaporated and purified ethylene glycol with a purity of ethylene glycol higher than 95%. The rest may be water and ethanol. The recover rate of the ethylene glycol is superior to 85%.

The initial liquid solution usually coming from a depolymerization process may contain contaminants, such as coloured contaminants, and other trace contaminants and components within the initial solution. Trace contaminants may include for example heavy metals, organic contaminants such as isophthalic acid, phthalic acid, and benzoic acid, aromatic compounds, odors, adhesives, and dyes. The contaminants may also, even if they are present only in small amounts, form a layer inside the distillation column, reducing the heat exchange efficiency and damaging the column on the long run. Thus, if such contaminants are present, it is preferred that the contaminants remain soluble in the solution before performing the second distillation step (c). For this, polyethylene glycol (PEG), which is a heavy component, is added either to the first solution before the first distillation step (b) and/or to the second solution containing ethylene glycol before the second distillation step (c), so that the at least one contaminant is kept soluble in the solution before performing the second distillation step (c). The heavy component added before the second distillation step (c) has to have the following criteria: organic liquid solvent, boiling point higher than 200°C, miscible with ethylene glycol.

In a preferred embodiment, if the initial liquid solution comprises at least one contaminant, the PEG is added before the second distillation step. In another preferred embodiment, the PEG is added before the first distillation step as well as before the second distillation step. In the second distillation step, the PEG forces the contaminants, especially the dyes, to be in the solution. The PEG is miscible with the organic contaminants, such as dyes, and keep them in a solution/liquid form. It does not chemically react with them. Together they form a one phase liquid solution. Thus, in the second distillation step, the rest of the second solution obtained on the bottom of the second distillation column contains PEG and the at least one contaminant, such as for example PEG and a dye.

In a further embodiment of the invention, if the initial liquid solution comprises at least one contaminant and PEG is added before the second distillation step, the method comprises an additional step of mixing the rest of the second solution on the bottom of the second distillation column after the second distillation step (d) with activated carbon to absorb the at least one organic molecule and to separate the polyethylene glycol (PEG) for reuse.

### Examples

The present invention will now be described in more detail with the aid of examples which, however, do not limit the scope of the subject matter of the invention.

The liquid phase after the PET depolymerization reaction as described in WO2020173961A1 is taken as the initial liquid solution and comprises water, ethanol, ethylene glycol (EG), dissolved sodium hydroxide (NaOH), contaminants such as dyes, and other organic contaminants such as for example isophthalic acid or phthalic acid. The EG content in the initial liquid solution is usually between 3 to 5 % by weight of the total liquid solution, while NaOH is between 8 to 11 % by weight of the total solution, Ethanol 65-75 % by weight of the total solution and water 13-15 % by weight of the total solution. The initial liquid solution is consequently alkaline (pH = 14).

### Example 1:

The initial liquid solution comprises 31g ethylene glycol, 650g ethanol and 205g water (the weight ration ethanol/water is 3.16), 4g NaOH and some contaminants, such as dyes. This initial liquid solution is pretreated using 40g sulfonic acidic resin as an ion exchange resin, where the sodium ion (salt) is removed and water produced. There is a production of 0.45g of water for each 1g of removed sodium ion. This results in neutralization of the solution to pH = 6-7, obtaining a first solution with 31g ethylene glycol, 651g ethanol and 206g water, and some contaminants, but no NaOH. This first solution is then mixed with 15g PEG (polyethylene Glycol), a heavy component that will force the contaminants to be in solution.

In a first distillation step, the first solution is distilled at 110-120°C and 647.5g ethanol and 205g water is evaporated on the top of the first distillation column, and on the bottom of the first distillation column a second solution is obtained containing 30g ethylene glycol, 3.4g ethanol and 1.1g water, 15g PEG and some contaminants.

In the second distillation step, the second solution is distilled at 200-290°C and 29.5g purified ethylene glycol is obtained on the top of the second distillation column containing a maximum of 0.12g water and 0.38g ethanol. This process provides a purity of EG higher than 95% and recover rate superior to 85%.

On the bottom of the second distillation column the rest of the second solution containing 0.5g ethylene glycol, 0.96g water and 3.04g ethanol, 15g PEG and some contaminants is obtained. The remaining solution is mixed with 1g of activated carbon and mixed under agitation for 30 mins. After 30 mins, the solution is filtered using filter paper and the activated carbon, which has already adsorbed the dyes, stays on the filter while the PEG solution is clear, without organic contaminants nor dyes, and is collected in a flask to be reused.

### Example 2:

In a second experiment, the same solutions are used, but the 15g PEG is added to the second solution before the second distillation step instead of to the first solution before the first distillation step:
The initial liquid solution comprises 31g ethylene glycol, 650g ethanol and 205g water (the weight ration ethanol/water is 3.16), 4g NaOH and some contaminants, such as dyes. This initial liquid solution is pretreated using 40g sulfonic acidic resin as an ion exchange resin, where the sodium ion (salt) is removed and water produced. There is a production of 0.45g of water for each 1g of removed sodium ion. This results in neutralization of the solution to pH = 6-7, obtaining a first solution with 31g ethylene glycol, 651g ethanol and 206g water, and some contaminants, but no NaOH.

In a first distillation step, the first solution is distilled at 110-120°C and 647.5g ethanol and 205g water is evaporated on the top of the first distillation column, and on the bottom of the first distillation column a second solution is obtained containing 30g ethylene glycol, 3.4g ethanol and 1.1g water, and some contaminants. This second solution is then mixed with 15g PEG (polyethylene Glycol), a heavy component that will force the contaminants to be in solution.

In the second distillation step, the second solution is distilled at 200-290°C and 29.5g purified ethylene glycol is obtained on the top of the second distillation column containing a maximum of 0.12g water and 0.38g ethanol. This process provides a purity of EG higher than 95% and recover rate superior to 85%.

On the bottom of the second distillation column the rest of the second solution containing 0.5g ethylene glycol, 0.96g water and 3.04g ethanol, 15g PEG and some contaminants is obtained. The remaining solution is mixed with 1g of activated carbon and mixed under agitation for 30 mins. After 30 mins, the solution is filtered using filter paper and the activated carbon, which has already adsorbed the dyes, stays on the filter while the PEG solution is clear, without organic contaminants nor dyes, and is collected in a flask to be reused.

## Claims

1. A method of extracting ethylene glycol from an initial liquid solution comprising water, ethanol, ethylene glycol (EG), and at least one salt, the method comprising the steps of:
a. removing the at least one salt from the initial liquid solution using an ion exchange resin obtaining a first solution;
b. performing a first distillation step in a first distillation column with the first solution at a temperature between 100-180 °C, extracting water and ethanol on top of the first distillation column and obtaining a second solution containing ethylene glycol and the rest of the first solution on the bottom of the first distillation column; and
c. performing a second distillation step in a second distillation column with the second solution containing ethylene glycol at a temperature between 200-290 °C obtaining purified ethylene glycol on the top of the second distillation column and the rest of the second solution on the bottom of the second distillation column.

2. The method of claim 1, wherein the initial liquid solution originates from a waste source such as the liquid solution coming from a PET depolymerization process, and wherein the initial liquid solution comprises at least one contaminant.

3. The method of claim 2, wherein polyethylene glycol (PEG) is added to the first solution before the first distillation step (b) and/or (preferably or) to the second solution containing ethylene glycol before the second distillation step (c), so that the at least one contaminant is kept soluble in the solution before performing the second distillation step (c).

4. The method of any one of the preceding claims, wherein the ion exchange resin is a strong cationic resin, preferably sulfonic acidic resin.

5. The method of any one of the preceding claims, wherein the at least one salt is sodium hydroxide (NaOH).

6. The method of any one of claims 2 to 5, wherein the at least one contaminant is a dye.

7. The method of any one of the preceding claims, wherein the temperature of the first distillation step in step (c) is between 110 and 120 °C.

8. The method of any one of the preceding claims, wherein the pH of the initial liquid solution is neutralized from 14 to between 6 and 7 in step (a).

9. The method of any one of claims 3 to 8, wherein the method comprises an additional step of mixing the rest of the second solution on the bottom of the second distillation column after the second distillation step (d) with activated carbon to absorb the at least one contaminant and to separate the polyethylene glycol (PEG) for reuse.
